# EUROPEAN PATENT APPLICATION

(11) **EP 1 780 221 A1**
(43) Date of publication of application: **02.05.2007**
(21) Application number: 05758020.1
(22) Date of filing: 04.07.2005
(51) Int. Cl.: C07K 16/40, A61K 39/395, A61P 19/02, A61P 29/00, A61P 35/00, C12N 5/10, C12N 9/99, C12N 15/02, C12P 21/08, C12Q 1/02, G01N 33/53

(54) **ANTI-SYNOVIOLIN ANTIBODY**

(30) Priority: 02.07.2004 JP 2004197010
(71) Applicant: Locomogene, Inc., Kanagawa 230-0046 (JP)
(72) Inventor: NAKAJIMA, Toshihiro, Yokohama-shi, Kanagawa 224-0001 (JP); YAMASAKI, Satoshi, Tokyo 105-0001 (JP); ZHANG, Lei, Tokyo 105-0001 (JP); AMANO, Tetsuya, Kawasaki-shi, Kanagawa 214-0037 (JP)
(74) Representative: Woods, Geoffrey Corlett
(86) International application number: PCT/JP2005/012706
(87) International publication number: WO 2006/004207

(57) **Abstract**

The present invention relates to an antibody against synoviolin or a fragment thereof for providing a monoclonal antibody capable of recognizing a part of synoviolin, which monoclonal antibody is capable of inhibiting the auto-ubiquitination of synoviolin.

SEQUENCE LISTING

<110> Locomogene Inc.

<120> Anti-Synoviolin antibody

<130> G06-0076

<150> JP2004-197010
<151> 2004-07-02

<160> 5

<170> PatentIn version 3.3

<210> 1
<211> 3374
<212> DNA
<213> Homo sapiens

<220>
<221> CDS
<222> (403)..(2256)

<400> 1
gccctttctt atgagcatgc ctgtgttggg ttgacagtga gggtaataat gacttgttgg 60
ttgattgtag atatagggct ctcccttgca aggtaattag gctccttaaa ttacctgtaa 120
gattttcttg ccacagcatc cattctggtt aggctggtga tcttctgagt agtgatagat 180
tggttggtgg tgaggtttac aggtgttccc ttctcttact cctggtgttg gctacaatca 240
ggtggcgtct agagcagcat gggacaggtg ggtaagggga gtcttctcat tatgcagaag 300
tgatcaactt aaatctctgt cagatctacc tttatgtagc ccggcagtcg cgcggattga 360
gcgggctcgc ggcgctgggt tcctggtctc cgggccaggg ca atg ttc cgc acg 414
gca gtg atg atg gcg gcc agc ctg gcg ctg acc ggg gct gtg gtg gct 462
cac gcc tac tac ctc aaa cac cag ttc tac ccc act gtg gtg tac ctg 510
acc aag tcc agc ccc agc atg gca gtc ctg tac atc cag gcc ttt gtc 558
ctt gtc ttc ctt ctg ggc aag gtg atg ggc aag gtg ttc ttt ggg caa 606
ctg agg gca gca gag atg gag cac ctt ctg gaa cgt tcc tgg tac gcc 654
gtc aca gag act tgt ctg gcc ttc acc gtt ttt cgg gat gac ttc agc 702
ccc cgc ttt gtt gca ctc ttc act ctt ctt ctc ttc ctc aaa tgt ttc 750
cac tgg ctg gct gag gac cgt gtg gac ttt atg gaa cgc agc ccc aac 798
atc tcc tgg ctc ttt cac tgc cgc att gtc tct ctt atg ttc ctc ctg 846
ggc atc ctg gac ttc ctc ttc gtc agc cac gcc tat cac agc atc ctg 894
acc cgt ggg gcc tct gtg cag ctg gtg ttt ggc ttt gag tat gcc atc 942
ctg atg acg atg gtg ctc acc atc ttc atc aag tat gtg ctg cac tcc 990
gtg gac ctc cag agt gag aac ccc tgg gac aac aag gct gtg tac atg 1038
ctc tac aca gag ctg ttt aca ggc ttc atc aag gtt ctg ctg tac atg 1086
gcc ttc atg acc atc atg atc aag gtg cac acc ttc cca ctc ttt gcc 1134
atc cgg ccc atg tac ctg gcc atg aga cag ttc aag aaa gct gtg aca 1182
gat gcc atc atg tct cgc cga gcc atc cgc aac atg aac acc ctg tat 1230
cca gat gcc acc cca gag gag ctc cag gca atg gac aat gtc tgc atc 1278
atc tgc cga gaa gag atg gtg act ggt gcc aag aga ctg ccc tgc aac 1326
cac att ttc cat acc agc tgc ctg cgc tcc tgg ttc cag cgg cag cag 1374
acc tgc ccc acc tgc cgt atg gat gtc ctt cgt gca tcg ctg cca gcg 1422
cag tca cca cca ccc ccg gag cct gcg gat cag ggg cca ccc cct gcc 1470
ccc cac ccc cca cca ctc ttg cct cag ccc ccc aac ttc ccc cag ggc 1518
ctc ctg cct cct ttt cct cca ggc atg ttc cca ctg tgg ccc ccc atg 1566
ggc ccc ttt cca cct gtc ccg cct ccc ccc agc tca gga gag gct gtg 1614
gct cct cca tcc acc agt gca gca gcc ctt tct cgg ccc agt gga gca 1662
gct aca acc aca gct gct ggc acc agt gct act gct gct tct gcc aca 1710
gca tct ggc cca ggc tct ggc tct gcc cca gag gct ggc cct gcc cct 1758
ggt ttc ccc ttc cct cct ccc tgg atg ggt atg ccc ctg cct cca ccc 1806
ttt gcc ttc ccc cca atg cct gtg ccc cct gcg ggc ttt gct ggg ctg 1854
acc cca gag gag cta cga gct ctg gag ggc cat gag cgg cag cac ctg 1902
gag gcc cgg ctg cag agc ctg cgt aac atc cac aca ctg ctg gac gcc 1950
gcc atg ctg cag atc aac cag tac ctc acc gtg ctg gcc tcc ttg ggg 1998
ccc ccc cgg cct gcc act tca gtc aac tcc act gag ggg act gcc act 2046
aca gtt gtt gct gct gcc tcc tcc acc agc atc cct agc tca gag gcc 2094
acg acc cca acc cca gga gcc tcc cca cca gcc cct gaa atg gaa agg 2142
cct cca gct cct gag tca gtg ggc aca gag gag atg cct gag gat gga 2190
gag ccc gat gca gca gag ctc cgc cgg cgc cgc ctg cag aag ctg gag 2238
tct cct gtt gcc cac tga cactgcccca gcccagcccc agcctctgct 2286
cttttgagca gccctcgctg gaacatgtcc tgccaccaag tgccagctcc ctctctgtct 2346
gcaccaggga gtagtacccc cagctctgag aaagaggcgg catcccctag gccaagtgga 2406
aagaggctgg ggttcccatt tgactccagt cccaggcagc catggggatc tcgggtcagt 2466
tccagccttc ctctccaact cttcagccct gtgttctgct ggggccatga aggcagaagg 2526
tttagcctct gagaagccct cttcttcccc cacccctttc caggagaagg ggctgcccct 2586
ccaagcccta cttgtatgtg cggagtcaca ctgcagtgcc gaacagtatt agctcccgtt 2646
cccaagtgtg gactccagag gggctggagg caagctatga acttgctcgc tggcccaccc 2706
ctaagactgg tacccatttc cttttcttac cctgatctcc ccagaagcct cttgtggtgg 2766
tggctgtgcc ccctatgccc tgtggcattt ctgcgtctta ctggcaacca cacaactcag 2826
ggaaaggaat gcctgggagt gggggtgcag gcgggcagca ctgagggacc ctgccccgcc 2886
cctcccccca ggcccctttc ccctgcagct tctcaagtga gactgacctg tctcacccag 2946
cagccactgc ccagccgcac tccaggcaag ggccagtgcg cctgctcctg accactgcaa 3006
tcccagcgcc caaggaaggc cacttctcaa ctggcagaac ttctgaagtt tagaattgga 3066
attacttcct tactagtgtc ttttggctta aattttgtct tttgaagttg aatgcttaat 3126
cccgggaaag aggaacagga gtgccagact cctggtcttt ccagtttaga aaaggctctg 3186
tgccaaggag ggaccacagg agctgggacc tgcctgcccc tgtcctttcc ccttggtttt 3246
gtgttacaag agttgttgga gacagtttca gatgattatt taatttgtaa atattgtaca 3306
aattttaata gcttaaattg tatatacagc caaataaaaa cttgcattaa caaaaaaaaa 3366
aaaaaaaa 3374

<210> 2
<211> 617
<212> PRT
<213> Homo sapiens

<400> 2

<210>  3
<211>  15
<212>  PRT
<213>  Homo sapiens

<400>  3

Ser Leu Ala Leu Thr Gly Ala Val Val Ala His Ala Tyr Tyr Cys

1                   5                   10                  15

<210>  4
<211>  15
<212>  PRT
<213>  Homo sapiens

<400>  4

Thr Cys Arg Met Asp Val Leu Arg Ala Ser Leu Pro Ala Gln Ser
1               5                   10                  15

<210>  5
<211>  15
<212>  PRT
<213>  Homo sapiens

<400>  5

Gly Ala Ala Thr Thr Thr Ala Ala Gly Thr Ser Ala Thr Ala Cys
1               5                   10                  15

## Description

### FIELD OF THE INVENTION

The present invention relates to an anti-synoviolin antibody. More specifically, the present invention relates to an antibody capable of inhibiting auto-ubiquitination of synoviolin, to a pharmaceutical compound comprising the antibody and to a method for detecting a cell expressing synoviolin by using the antibody.

### BACKGROUND OF THE INVENTION

Rheumatoid arthritis (hereinafter, simply referred to as "RA") is a systemic inflammatory disorder associated with an abnormal proliferation of synovial tissue in a joint. Synovial cells are fibroblast-like cells that form 1 to 6 epithelial-like layers in a synovial membrane of a joint, which are thought to supply proteoglycan and hyaluronic acid to the synovial fluid. In a joint of an RA patient, symptoms such as proliferation of the synovial tissue, a multilayer structure resulting from such proliferation, infiltration of the synovial cells to other tissues or the like can be observed. An autoantibody against an Fc region of auto-immunoglobulin (IgG) is present in serum from an RA patient. This autoantibody, also called an RA factor, has conventionally been utilized as a diagnostic index characteristic of RA.

The etiology of RA as one of the autoimmune diseases, however, is not yet well understood. For RA diagnosis based on detection of the RA factor, specificity to the disease or a system produced by the antibody is not yet understood and nor is the association between the RA factor and the etiology of the disease.

Conditions of RA can be viewed in two aspects, which are (a) different kinds of *in vivo* immune reactions and (b) proliferation of a synovial membrane of a joint associated with bone destruction. The former immune reactions have been well studied and their mechanisms at the molecular level are gradually becoming clear. As to the studies on the latter synovial cells in a joint, however, even the biologic property of the cell has not been understood although it is the principal of RA.

Thus, in order to understand conditions of RA, the present inventors have studied the underlying molecular mechanism of onset and development of chronic and intractable diseases. First, a cultured human synovial cell from an RA patient was used as an immunogen to obtain an anti-human synovial cell antibody for immunoscreening a cDNA library of the synovial cell. As a result, a novel gene expressed in synovial tissue of an RA patient was found. This gene was successfully isolated, the protein coded by this gene was named synoviolin after the tissue (i.e., synovial cell) expressing the gene, and the physiological meaning thereof was revealed (WO02/052007, pamphlet). Synoviolin found by the present inventors is closely related to abnormal proliferation of the synovial tissue that is a major cause of RA disorders and thus it is expected of providing very important information for diagnosis. Moreover, synoviolin is also known to code for E3 ubiquitin ligase having an RING finger motif as found based on a protein conformation prediction system. This motif plays an important role in ubiquitination of a protein. In fact, it is proved to have an auto-ubiquitination activity, and thus it is also predicted to have regulated functions of the protein.

The present inventors have pursued the research on signal transduction by synoviolin and found for the first time that synoviolin has a ubiquitin ligase activity that causes auto-ubiquitination.

### DISCLOSURE OF THE INVENTION

As described above, there has been a demand for developing an antibody for inhibiting auto-ubiquitination of synoviolin and a pharmaceutical composition comprising the antibody.

The present inventors have gone through keen study for solving these problems, as a result of which they have found an anti-synoviolin antibody that inhibits auto-ubiquitination of synoviolin based on the finding that synoviolin has a ubiquitin ligase activity that causes auto-ubiquitination, thereby accomplishing the present invention.

Thus, the present invention is as follows:
(1) An antibody or a fragment thereof against synoviolin, which is capable of inhibiting auto-ubiquitination of synoviolin. The antibody or the fragment thereof may not influence ubiquitination of the substrate protein of synoviolin. Furthermore, the antibody may be a monoclonal antibody.
(2) A monoclonal antibody against synoviolin or a fragment thereof produced by a hybridoma obtained by cell fusion between a myeloma cell and an antibody-producing cell derived from an animal immunized with a peptide having an amino acid sequence represented by any one of SEQ ID NOS:3-5 as an antigen, which is capable of inhibiting auto-ubiquitination of synoviolin.
(3) A hybridoma obtained by cell fusion between a myeloma cell and an antibody-producing cell derived from an animal immunized with a peptide having an amino acid sequence represented by any one of SEQ ID NOS:3-5 as an antigen, which produces a monoclonal antibody capable of inhibiting auto-ubiquitination of synoviolin.
(4) A method for producing a monoclonal antibody against synoviolin capable of inhibiting auto-ubiquitination of synoviolin, comprising: culturing a fusion cell between a myeloma cell and an antibody-producing cell derived from an animal immunized with a peptide having an amino acid sequence represented by any one of SEQ ID NOS:3-5 as an antigen; and collecting the monoclonal antibody from the resulting culture.
(5) A pharmaceutical composition comprising the antibody or the fragment thereof according to (1) or (2). This pharmaceutical composition may be used for treating or preventing, for example, cell proliferative diseases such as rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia and malignant lymphoma.
(6) An inhibitor for auto-ubiquitination of synoviolin, comprising the antibody or the fragment thereof according to (1) or (2).
(7) A reagent for detecting a cell containing synoviolin, comprising the antibody or the fragment thereof according to (1) or (2).
(8) A reagent for detecting a cell proliferative disease, comprising the antibody or the fragment thereof according to (1) or (2). Examples of the cell proliferative diseases include rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia and malignant lymphoma.
   The above-mentioned cell may be any one selected from the group consisting of synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell and apical ectodermal ridge.
(9) A method for inhibiting auto-ubiquitination of synoviolin, comprising reacting the antibody or the fragment thereof according to (1) or (2) with synoviolin.
(10) A method for detecting a synoviolin-expressing cell, comprising reacting the antibody or the fragment thereof according to (1) or (2) with a biologic sample.
(11) A method for detecting a cell proliferative disease caused by synoviolin, comprising reacting the antibody or the fragment thereof according to (1) or (2) with a biologic sample taken from a subject. The types of the cell proliferative disease and the cell are the same as listed above.

The cell may be any cell selected from the group consisting of synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell and apical ectodermal ridge.

All of the prior art publications cited herein are incorporated herein by reference.

### BRIEF DESCRIPTION OF THE DRAWINGS

Figure 1 shows a picture of the results from western blotting using SL-1 antibody performed on synovial cells derived from rheumatic arthritis patients (RA: 2 samples) and osteoarthritis patients (OA: 2 samples).
Figure 2 shows pictures of the results from fluorescent immunostaining using SL-1 antibody performed on synovial cells derived from an RA patients.
Figure 3 is a view showing the results from immunostaining using SL-1 antibody performed on synovial tissue derived from an RA patients and images of hematoxylin-eosin (HE) staining.
Figure 4 shows pictures of western blotting analysis showing that auto-ubiquitination of MBP-dTM Syno-His fusion protein was inhibited by SL-1 antibody.
Figure 5 shows pictures of western blotting analysis showing that ubiquitination of synoviolin of GST-P4HA1 fusion protein was unaffected with SL-1 antibody.

### BEST MODES FOR CARRING OUT THE INVENTION

Hereinafter, the present invention will be described in more detail.

### 1. General

An antibody of the invention is an antibody against synoviolin that is capable of inhibiting auto-ubiquitination of synoviolin and that is obtained by immunization using, as an antigen, a peptide having a part of the amino acid sequence of the RING finger domain of synoviolin.

Ubiquitination is a process in which enzymes such as ubiquitin-activating enzyme (E1), ubiquitin-conjugating enzyme (E2) and ubiquitin ligase (E3) cooperate to successively bind ubiquitin to a substrate protein. The physiological significance of ubiquitination has conventionally been recognized as a tag modification for transportation to the proteasome system, i.e., a protein degradation mechanism. From subsequent studies, the significance of ubiquitination is currently characterized as a reversible protein modification system for controlling protein functions.

Herein, the term "self-ubiquitination" or "auto-ubiquitination" means that synoviolin having a ubiquitin ligase activity becomes the substrate protein for ubiquitination by itself and binds ubiquitin without depending on other ubiquitin ligase. Auto-ubiquitination of synoviolin became apparent when the present inventors found that synoviolin had a ubiquitin ligase activity. Specifically, overexpression of synoviolin in a mouse results in spontaneous arthropathy associated with proliferation of synovial cells (WO02/052007, pamphlet) (Amano T, et al., Genes Dev. 17(19):2436-49, 2003). On the other hand, an RING finger motif of E3 ubiquitin ligase is an active center of the enzyme and the enzyme activity is totally deactivated with a substitution of only a single amino acid in that motif. In fact, a mouse expressing a single amino-acid mutant (C307S) of the enzyme activity center of synoviolin does not present arthritis (Amano. T. et al., *supra).* This indicates that auto-ubiquitination can be important for functional expression of synoviolin and that auto-ubiquitination of synoviolin is the representative function of synoviolin. This auto-ubiquitination is not only observed in a full-length molecule of endogenous synoviolin but also found to occur with only a tag protein-fused intracellular part of synoviolin. Based on these findings, the present inventors have gone through keen study, thereby accomplishing the present invention.

According to the present invention, the term "antibody" refers to the whole antibody molecule (either polyclonal antibody or monoclonal antibody) capable of binding to synoviolin or a fragment thereof as an antigen, and further includes a fragment thereof, which is an active fragment having an antigen-antibody reaction activity, specifically, Fab, F(ab')₂, Fv, recombinant Fv and single-strand Fv.

### 2. Synoviolin

(1) Synoviolin and equivalent protein thereof
   Other than synoviolin itself, a similar or equivalent protein, a fragment thereof or a peptide fragment thereof can be used as an immunogen in order to obtain an antibody against synoviolin. Any of them can act as an antigen to anti-synoviolin antibody. This is because, an antibody is generally derived via recognition by a small region on the surface of the protein known as an epitope site rather than comprehensive recognition by the whole protein structure as an antigen. Such an epitope site is formed with consecutive or intermittent parts of a polypeptide chain. Therefore, for one type of antibody against synoviolin, a site that acts as an epitope corresponds to a limited peptide site of synoviolin.
   The source of synoviolin used with the present invention is not particularly limited and may include human, mouse and rat. Synoviolin used may be, for example, a polypeptide having the amino acid sequence represented by SEQ ID NO:2.
   A protein similar or equivalent to synoviolin may also be used as an antigen (as described later).
(2) Preparation from biomaterial
   Synoviolin may be obtained from synovial tissue from an RA patient. Since synovial cells can be *cultured in vitro,* synoviolin may be collected from this culture. Specifically, synovial cells are isolated from synovial tissue or the like that has been surgically excised from an RA patient by synovectomy. The isolated cells are cultured so as to collect synovial cells as adherent cells (Nakajima T., et al., J. Clin. Invest. 92:186-193, 1993). From the collected cells, synoviolin is extracted and purified by combining known protein purification techniques. The resulting synoviolin or a fragment thereof can be used as an immunogen for obtaining an antibody.
(3) Preparation by genetic engineering process
   Synoviolin is not only obtained from a biomaterial but may also be obtained as an expression product of a gene recombinant by integrating a gene coding for synoviolin into an appropriate expression system.
   The polynucleotide coding for synoviolin may be derived from any source. Thus, other than genomic DNA and cDNA, the polynucleotide may be obtained by synthesis. The nucleic acid may be either DNA or RNA. As long as they code for synoviolin, polynucleotides having any of nucleotide sequences based on degeneracy of genetic codes or polynucleotides including any of modified nucleotides are also comprised.
   Polynucleotides coding for synoviolin can be isolated by cloning a cDNA library obtained based on mRNAs extracted from synovial cells from an RA patient. Specifically, a desired polynucleotide can be obtained by amplifying a cDNA library by PCR or the like and screening the hybridized clones (Short J.M, et al., Nucleic Acid Res. 16:7583-7600, 1988). SEQ ID NO:1 represents a nucleotide sequence of DNA coding for the polypeptide represented by SEQ ID NO:2. Examples of preferable host/vector systems for integrating a polynucleotide coding for synoviolin into an appropriate expression system include expression vector pGEX and *E.coli.*
   Other than those mentioned above, in the case where a bacterium is used as a host, expression vectors for fusion proteins utilizing various tags such as a histidine tag, an HA tag and a FLAG tag are commercially available. An expression system utilizing *Pichia* yeast or an expression system utilizing baculovirus vector that uses an insect cell as a host are useful as a host/vector system for expressing a protein with a sugar chain. Moreover, a mammal cell has been utilized for transfection of vectors utilizing a promoter such as cytomegalovirus (CMV) promoter, Rous sarcoma virus (RSV) promoter or SV40.
   According to the present invention, synoviolin can also be collected from the synoviolin gene or the vectors described above. Thus, according to the present invention, a cell-free protein synthesis system can be employed without using any live cell to produce synoviolin.
   A cell-free protein synthesis system is a system for synthesizing a protein in an artificial vessel such as a test tube using a cell extract. The cell-free protein synthesis system used with the present invention also includes a cell-free transcription system that synthesizes RNA using DNA as a template.
   In this case, organisms corresponding to the above-described host are the sources of the cell extract described below. Herein, the cell extract used may be an extract derived from an eukaryotic cell or a prokaryotic cell, for example, extracts of wheat germ, rabbit blood reticulocyte, mouse L-cell, HeLa cell, CHO cell, budding yeast or *E. coli.* These cell extracts may be in a concentrated form or in an unconcentrated form.
   The cell extract may be obtained by, for example, ultrafiltration, dialysis, polyethylene glycol (PEG) precipitation or the like. Moreover, according to the present invention, the cell-free protein synthesis can be carried out using a commercially available kit. Examples of such kits include reagent kit PROTEIOS^{™} (Toyobo), TNT^{™} System (Promega), synthesizer PG-Mate^{™} (Toyobo) and RTS (Roche Diagnostics).
   Synoviolin obtained by the above-described cell protein synthesis may be purified by selecting an appropriate chromatography as described above.
(4) "Functionally equivalent protein" to synoviolin
   As protein that can be an antigen upon preparing an anti-synoviolin monoclonal antibody not only comprises human synoviolin extracted from synovial cell but also comprises various proteins that are functionally equivalent to synoviolin. Such proteins may be either artificial or natural and comprise a mutant protein in which one or several amino acids have been substituted, deleted, added and/or inserted in an amino acid sequence of human synoviolin, a modified protein whose amino-acid side chain has been modified, and a fusion protein fused with other protein.
   The number or sites of mutation or modification of amino acids in these proteins and is not limited as long as the functions of synoviolin are maintained. For example, a mutant protein in which one or more (e.g., one or several) amino acids have been substituted, deleted, added and/or inserted in the amino acid sequence represented by SEQ ID N0:2, or a modified protein whose amino-acid side chain or the like has been modified may be used.
   Specifically, a mutant synoviolin polypeptide having the following amino acid sequence and having the equivalent activity to synoviolin described above can be used:
   (i) an amino acid sequence having one or more (preferably one or several (e.g., 1 to 10, more preferably 1 to 5)) amino acids deleted in the amino acid sequence represented by SEQ ID NO:2;
   (ii) an amino acid sequence having one or more (preferably one or several (e.g., 1 to 10, more preferably 1 to 5)) amino acids in the amino acid sequence represented by SEQ ID NO:2 substituted with other amino acids;
   (iii) an amino acid sequence having one or more (preferably one or several (e.g., 1 to 10, more preferably 1 to 5)) amino acids added to the amino acid sequence represented by SEQ ID NO:2; or
   (iv) an amino acid sequence having a combination of (i) to (iii) above.

   The polypeptide used with the invention may be one having homology with the above amino acid sequence of synoviolin as long as it has the same function as synoviolin. Examples include amino acid sequence having about 85% or more, preferably about 90% or more, more preferably about 95% ore more homology with the above amino acid sequence of the synoviolin polypeptide.
   A polynucleotide coding for an amino acid sequence having one or more amino acids deleted, inserted or added in the amino acid sequence represented by SEQ ID NO:2 may be prepared according to a method such as a site-directed mutagenesis described, for example, in "Molecular Cloning, A Laboratory Manual 2nd ed." (Cold Spring Harbor Press (1989)), "Current Protocols in Molecular Biology" (John Wiley and Sons 1987-1997), and Kunkel T.A., Proc. Natl. Acad. Sci. USA 82: 488-92, 1985.
   Mutation can be introduced into a polynucleotide according to a known process such as Kunkel method or Gapped duplex method using a mutation introducing kit utilizing a site-directed mutagenesis such as QuikChange^{™} Site-Directed Mutagenesis Kit (Stratagene), GeneTailor^{™} Site-Directed Mutagenesis System (Invitrogen) and TaKaRa Site-Directed Mutagenesis System (Mutan-K, Mutan-Super Express Km, etc.: Takara Bio Inc.).
   First, the "functionally equivalent protein" described above may be a protein that is immunologically equivalent to synoviolin. Thus, a protein functionally equivalent to synoviolin comprises a domain of synoviolin and that reacts with the antibody present in serum of an RA patient that specifically recognizes synoviolin.
   Secondly, a protein functionally equivalent to synoviolin is also defined based on the binding property with a ligand protein that binds with synoviolin. Examples include HMG-CoA Reductase Degradation 3 (Hrd3), procollagen-proline, 2-oxoglutarate 4-dioxygenase (proline 4 hydraxylase), α polypeptide I (P4HA1) and Homocysteine-inducible endoplasmic reticulum stress-inducible ubiquitin-like domain member 1 (Herp) (Japanese Patent Applications Nos. 2003-295951, 2004-076931 and 2003-350704).
   Thirdly, a protein functionally equivalent to human synoviolin includes a protein having an activity of promoting outgrowth of synovial membrane. In transgenic mice introduced with human synoviolin gene, swelling of fingers associated with arthritis were frequently observed and, in terms of histology, bone destruction associated with outgrowth of synovial membrane and abnormal bone neoplasm were observed in the joints of these fingers.
   Fourthly, a protein functionally equivalent to human synoviolin includes a protein having an activity contributing to normal bone formation or development of limbs. Synoviolin is strongly expressed upon generation in sites where bones or cartilages such as parietal bone, limbs and ears are formed, and strong expression is observed in Apical Ectodermal Ridge (AER), cartilage and bone anlage during the limb forming phase.
   Fifthly, a protein functionally equivalent to synoviolin of the present invention may also be defined based on biochemical activities of synoviolin, for example, ubiquitin ligase activity. These biochemical activities are proven by various motifs found in synoviolin and experimental results.
(5) Synoviolin fusion protein or modified protein
   A protein functionally equivalent to synoviolin also comprises proteins with various modifications such as modification, conservative substitution, deletion, addition or insertion of an amino acid residue, physiological or man-caused modification of, for example, sugar chain, and fusion with a label such as fluorescent or radioactive material or other protein. For example, in the gene recombinant described above, modifications of the sugar chain may differ according to the host used for expression. Even when the modifications of the sugar chain differ, however, they are all synoviolin or proteins functionally equivalent to synoviolin as long as they have the equivalent aspect to synoviolin.

An example of a fusion protein with other protein includes a protein that is added with an additional amino acid sequence such as a FLAG tag, an HA tag or a histidine tag and that maintains at least one of the aspects as the protein functionally equivalent to synoviolin. In addition, a fusion protein maintaining at least one of the functions of synoviolin is also included even if it has a different activity from that of synoviolin.

Since synoviolin is capable of auto-ubiquitination only with its intracellular domain, synoviolin used with the present invention may be synoviolin dTM lacking the cell transmembrane domain. For example, MBP-synoviolin dTM-His in which the above tag proteins are fused to the dTM may equally be used with the present invention.

Alternatively, a fusion protein maintaining at least one of the functions of synoviolin is also included even if it has a different activity from that of synoviolin.

### 3. Preparation of antigen

As an antigen for preparing an anti-synoviolin antibody, synoviolin or a protein functionally equivalent to synoviolin can be used as described above. In detecting an antibody, however, not only an antigen molecule itself (i.e., synoviolin or a protein functionally equivalent to synoviolin) but also a complex including a fragment, a peptide fragment or a chemically-synthesized oligopeptide of these proteins and an appropriate carrier is often used as an antigen. This is because employing an analysis system that is specific to a predominant epitope or an epitope with some sort of clinical meaning can avoid influence of non-specific reactions. In other words, hybridoma cloning and selection of clones described later can be performed efficiently and further an anti-synoviolin monoclonal antibody with a high antibody titer can be obtained. Preferably, a fewer but certain (maybe lower-order) structures are recognized as epitope sites rather than a large number of domains in a high-order structure.

This approach is also effective in the case of obtaining a monoclonal antibody having a higher specificity and affinity to synoviolin. Specifically, a domain that functions as an epitope can be determined based on a method for obtaining an immunologically active domain peptide described below.

It is known that an epitope may consist of at least 3 amino acid residues. Immunological identification from other proteins is said to be possible with at least 8 amino acid residues. Therefore, a fragment that has 8 consecutive amino acid residues, usually 9 amino acid residues, preferably 10 amino acid residues and more preferably 11-15 amino acid residues selected from the amino acid sequence of synoviolin or a protein equivalent thereto and that specifically reacts with an antibody in patient's serum is desirable as an antigen for detecting an antibody in the present invention.

Furthermore, a method for enhancing immunoreactivity by adding various modifications to an oligopeptide making up the epitope is known to those skilled in the art. For example, a modification such as addition of an inactive protein (e.g., human serum albumin) or a meaningless amino acid sequence can contribute to enhancement of an immunoreactivity.

A fragment of synoviolin can be obtained by enzyme digestion using protease such as trypsin, chymotrypsin and pepsin or chemical cleavage using cyanogen bromide, or a combination thereof. The peptide of interest may be separated and purified from the produced peptide mixture by employing a known separation technique such as chromatography and electrophoresis.

According to an alternative method, DNA coding for synoviolin is randomly cleaved and the resultant is inserted into a phage vector to produce phage libraries presenting a domain peptide. These libraries can be subjected to immunoscreening with an antibody that recognizes synoviolin to determine an immunologically active domain.

From the strategic point of view described above, first, a presumably preferable peptide portion having 14 or more amino acid residues is determined by a method for analyzing the hydrophilic profile of synoviolin and other antigenic index. These peptides as candidate immunogens can be synthesized by a peptide synthesis technique by a known liquid-phase or solid-phase method. A solid-phase synthesis method as typified by Merrifield method can be carried out conveniently and in a short time.

As an α-amino protective group, tert-butyloxycarbonyl (Boc) is normally used, although 9-fluorenyl methoxy carbonyl (Fmoc) group developed by Sheppard et al. (Atherton, E and Sheppard, R.C, J.Chem. Soc. Chem. Comm. 165, 1985) is also available (see Nobuo Izumiya et al., "Fundamentals and Experiments of Peptide Synthesis", pp. 194-233, Maruzen, 1985).

According to the present invention, an automated peptide synthesizer based on a solid-phase synthesis method may be utilized. A synthesized peptide is separated from the resin in the presence of, for example, trifluoroacetic acid and then purified by reversed-phase high performance liquid chromatography. Preferably, the purified peptide has a purity of 85% or more.

Among these candidate peptides, peptides positive in detective reaction with an enzyme-labeled anti-IgG antibody following reaction between polyclonal antibodies collected from immunosensitized animals and synoviolin are used as immunizing antigens for producing the antibody of the invention.

An example of synoviolin fragment particularly useful as immunogens includes at least one peptide including the following amino acid sequences:
Syno-P3 (SLALTGAVVAHAYYC/SEQ ID N0:3);
Syno-P2 (TCRMDVLRASLPAQS/SEQ ID NO:4); and
Syno-P 1 (GAATTTAAGTSATAC/SEQ ID NO:5).

Immunogens prepared by binding these peptides with carrier proteins are specific to synoviolin and give an antibody having a sufficient binding affinity. The peptide of synoviolin useful as an immunogen is bound to Keyhole Limpet hemocyanin (KLH) as a carrier protein prior to sensitizing an animal.

Other carrier substances that can be used for obtaining an immunogen include purified tuberculin protein derivatives, tetanus toxoid, cholera toxin and B subunit thereof, diphtheria toxin, ovalbumin, bovine serum albumin, soybean trypsin inhibitor, muramyl dipeptide and Brown's lipoprotein). Reactions and reagents for binding a peptide with a carrier protein is described in known publications (for example, Shinobu Ohmi et al., Supp. of Cell Engineering, Laboratory Protocol Series, Anti-peptide Antibody Laboratory Protocols (New Edition), "From identification of gene product to analysis of protein functions", Shujunsha, 1994; and Coligan J.E. et al., CURRENT PROTOCOLS IN IMMUNOLOGY, Vol. 1,p. 9.4.1-9.4.11, 1991).

### 4. Immunization

(1) Preparation of polyclonal antibody against synoviolin
   The antigen prepared as described above is administered to mammals. The mammals are not particularly limited and are, for example, rats, mice or rabbits, preferably rabbits.
   A dose of the antigen given per animal in the case of rabbit is 1-10 mg without an adjuvant and 0.1-1 mg with an adjuvant. Examples of adjuvants include Freund's complete adjuvant (FCA), Freund's incomplete adjuvant (FIA) and aluminum hydroxide adjuvant. Immunization is mostly performed by intravenous, subcutaneous, intraperitoneal infusion or the like. The interval between the immunizations is not particularly limited and is a few days to a few weeks, preferably 2-5 weeks, which immunizations are performed for 1-10 times, preferably 2-5 times. From day 6 through day 60 following the day of final immunization, antibody titers are determined by enzyme immunoassay (ELISA (enzyme-linked immunosorbent assay) or EIA (enzyme immunoassay)), radioimmunoassay assay (RIA) or the like. Blood is drawn on a day that gave the highest antibody titer to obtain an antiserum.
   Subsequently, reactivity of the polyclonal antibody in the antiserum against the above-described proteins is determined by ELISA method or the like.
(2) Preparation of monoclonal antibody against synoviolin
   (i) Collection of antibody-producing cells
      In order to prepare a monoclonal antibody, synoviolin as an immunizing antigen, an immunologically equivalent protein thereof, or a fragment or a peptide fragment thereof is administered as an immunogen alone or with a carrier and a diluting solution to an antibody-producible site of a mammal. In this regard, an adjuvant may be added before the administration in order to enhance the antibody-producing ability (Adv. Tubercl. Res., 1:130-148, 1956). Examples of adjuvants include FCA, FIA and aluminum hydroxide adjuvant.
      A monoclonal antibody can be obtained as follows: forming a fusion cell between an immunocompetent cell (specifically, an antibody-producing cell) and a myeloma cell; cloning the cell; and selecting a clone that produces an antibody specific to synoviolin (Kohler, G. and Milstein, C, Nature 256: 495-7, 1975).
      For immunization, a complex containing the above-described peptide (or synoviolin, an immunologically equivalent protein thereof or a fragment thereof) as an immunogen is used, for example, in 20 µg to 1 mg for a single dose by dissolving in or mixing with an appropriate adjuvant for immunosensitization of an animal to be immunized.
      Examples of mammals to be immunized include mice, guinea pigs, rabbits, rats, sheep, goats, monkeys and dogs. Usually, mice or rabbits are preferably used for easy operation. Preferably, the antibody-producing cell and the myeloma cell are derived from animals of the same species. Administration is usually conducted once in every 2-6 weeks during a period of 3-6 months for about 2-10 times.
      The antibody-producing cells are collected by selecting individuals showing antibody titers among the animals immunized with the antigen and then spleen cells, lymph node cells or B lymphocytes are collected on 2-5 days after the final immunization. The antibody-producing cells contained in the above cells and myeloma cells having a self-propagating ability are fused. Clones that produce an anti-synoviolin monoclonal antibody of the invention are selected from the resulting hybridomas, thereby preparing hybridomas that produce the monoclonal antibody. Basically, this procedure may be carried out according to Kohler method ("Immunological Method", Academic Press, New York, 391, 1979).
   (ii) Cell fusion
      Fusion process may be carried out according to a known technique such as Kohler or Milstein method mentioned above. Examples of myeloma cells include cell strains derived from mouse myeloma such as P3U1, NS-1 and SP2/0 and mutant strains thereof, while P3U1 is particularly preferable. As a fusion promoter, polyethyleneglycol (PEG), Sendai virus or the like, preferably PEG, is used. Fusion is performed by suspending the myeloma cells in a medium such as a serum-free RPMI1640 medium, to which a solution containing antibody-producing cells such as the spleen cells prepared above is added and allowed to stand still for a while. After a brief centrifugation, the cells are collected, added with an RPMI1640 medium containing PEG and incubated at 37°C for 2-4 minutes, thereby completing cell fusion.
      The hybridomas collected by centrifuging the reaction product are transferred to a HAT (hypoxanthine, aminopterin, thymidine) medium, dispensed, for example, into a 96-well microplate and cultured for a predetermined period of time. Generally, the culture takes place in an incubator under 5% carbon dioxide gas at 20-40°C, preferably at 34-38°C. Usually, hybridomas are grown in a week and colony formation should be observed. The culture period is usually 5 days to 3 weeks, preferably 1-2 weeks.
   (iii) Antibody screening and cloning
      In general, monoclonal antibodies can be sorted in an animal cell culture media supplemented with HAT. Media used for sorting and breeding are not particularly limited as long as hybridomas can grow in them. For example, a serum-free medium for hybridoma culture, an RPMI medium supplemented with fetal calf serum (FCS), a GIT medium or the like can be used.
      The antibody titers of the culture supernatants in the wells that showed proliferation against the peptides are determined by an enzyme-antibody technique or the like, and the hybridomas are cloned, for example, by a limiting dilution method to obtain clones of the hybridomas of the invention.
      The formed hybridomas of the invention are usually screened as follows. A convenient method comprises: adding the supernatant of the hybridoma culture to a solid phase such as a microplate that is adsorbed with the peptide as the immunogen directly or together with a carrier; adding an anti-immunoglobulin antibody or protein A labeled with a radioactive substance, an enzyme or the like; and detecting the monoclonal antibody bound to the solid phase. Alternatively, the supernatant of the hybridoma culture may be added to a solid phase that is adsorbed with an anti-immunoglobulin antibody or protein A, and then a labeled antigen is added to detect the monoclonal antibody bound to the solid phase.
      Positive wells are selected following the above procedure. After a few days, the cells are seeded on a single 96-well plate per strain at, for example, 100 cells/plate and cultured for 10-14 days. Colonies are confirmed and the culture supernatants are applied to the screening plate having antigen solid phased thereon to examine in the same manner. The selected colonies are cultured, re-cloned, cultured for 10-14 days, and subjected to the same procedures of colony confirmation and examination of the culture supernatants. Wells are selected by parent strain and cultured in a 24-well plate. Supernatants are collected to check the clones for examining antibody subclasses and antibody production.
   (iv) Production and separation of monoclonal antibody
      The clone SL-1 sorted as described above is *cultured in vivo* or *in vitro* to produce the monoclonal antibody of the invention, i.e., SL-1 antibody. For this cultivation, methods conventionally employed in the art can be used.
      When the hybridoma SL-1 is cultured in an *in vitro* culture medium, SL-1 antibody can be separated from that culture. Enhancements of the growth rate and the antibody-producing efficiency require selection of the type of the medium, maintenance of the medium and control of the culture conditions (e.g., oxygen level in the medium, speed of agitation and contamination).
      When clones are cultured utilizing non-human warm-blooded animals, the monoclonal antibody is collected from their ascitic fluids and/or blood. For example, clones are seeded on an RPMI1640 medium supplemented with fetal calf serum to a predetermined cell density and cultured in an incubator at 37°C in the presence of 5% carbon dioxide gas. Then, the culture is inoculated intraperitoneally to the mice that have been pre-administered with pristine and the mice are bred for a predetermined period in a conventional fashion. Usually, following 1-2 weeks, ascitic fluids are taken to which ammonium sulfate is added to give salt precipitation. The monoclonal antibody is separated and purified from the resulting fraction by chromatography or the like.
      A method for isolating the monoclonal antibody from culture, ascitic fluids and/or blood is the same as a general method for purifying a polyclonal antibody, which employs a separation/purification method for immunoglobulin. Specifically, the following methods may be employed in an appropriate combination: salting-out, dialysis, filtration, enrichment, alcohol precipitation, isoelectric precipitation, various types of electrophoreses adsorption-desorption with an ion exchanger (e.g., DEAE resin), ultracentrifuge, gel filtration, specific affinity purification and else. The produced monoclonal antibody is subsequently enriched, dried and made into liquid or solid according to use.
   (v) Humanized or human antibody

   According to the present invention, an antibody comprises a humanized or human antibody.
   A human antibody can be prepared in the same manner as a usual monoclonal antibody by immunizing a mammal whose immune system has been exchanged for a human immune system.
   A humanized antibody is an antibody having a human type constant region and a partially human type variable region where the variable region has been reconstituted with a human-derived framework region (FR) and a mouse-derived complementarity determining region (called CDR). In order to prepare a humanized antibody, CDR is grafted from a variable region of a mouse antibody to a human variable region, and then this reconstituted human type variable region is fused with a human constant region. A method for preparing a humanized antibody can be realized by a genetic engineering technique and has been established in the art (Kazuhisa Sugimura, "Antibody medicine gains momentum; All about antibody engineering and antibody drug", Bioventure Vol.2, No.4, Yodosha, 2002).
(3) Characteristics of the antibody of the invention
   (i) It can inhibit auto-ubiquitination of synoviolin.
      Thus, it can be used for treating diseases caused by auto-ubiquitination of synoviolin.
   (ii) It does not influence ubiquitination of substrate protein P4HA1 of synoviolin
      Thus, it has no influence on physiological functions involving ubiquitination of P4HA 1.
   (iii) Isotype of the antibody of the invention is IgG1.

### 5. Application of the monoclonal antibody of the invention

The antibody of the invention (e.g., SL-1 antibody) can be utilized in various applications since it can specifically recognize synoviolin or a peptide fragment thereof. Hereinafter, some aspects of typical applications will be described taking SL-1 antibody as an example.
(1) Inhibition of auto-ubiquitination reaction of synoviolin
   Ubiquitination is a process in which ubiquitin-activating enzyme (E1), ubiquitin-conjugating enzyme (E2) and ubiquitin ligase (E3) cooperate to successively bind ubiquitin to a substrate protein. As described above, auto-ubiquitination of synoviolin means that synoviolin having a ubiquitin ligase activity becomes the substrate protein for ubiquitination by itself and binds ubiquitin by itself without depending on other ubiquitin ligase.
   The above enzymes such as ubiquitin-activating enzyme (E1) and ubiquitin-conjugating enzyme (E2) are commercially available and may be used appropriately. E1 is used in a range of 1-50 ng, preferably 20-40 ng and E2 is used in a range of 0.1-0.5 µg, preferably 0.2-0.3 µg per well of a 96-well microplate.
   As a low molecular weight reactive factor required for ubiquitination, Mg salts such as MgC1₂ and MgSO₄, ATP, EDTA, NAF, DTT (dithiothreitol), okadaic acid or the like may appropriately be selected for use. These compounds are also commercially and readily available.
   Moreover, a buffer for dissolving the enzymes or reagents, a washing buffer, a buffer used for determination, a buffer used to terminate the reaction and the like may be any known buffer (e.g., Tris-HCl) as long as it does not deactivate the enzymes or interfere with the reactions.
   His-tag-fused protein MBP-dTM Syno-His is used and mixed with the monoclonal antibody prepared above, an anti-FLAG antibody or mouse IgG to perform antigen-antibody reaction at 4°C for 1.5 hours.
   Then, auto-ubiquitination reaction takes place. The auto-ubiquitination reaction of synoviolin may be carried out under the following conditions. To a given amount of buffer (pH 6-8) as a reaction solvent, the above-mentioned predetermined amounts of reaction substances (other than synoviolin or its active derivative) required for auto-ubiquitination of synoviolin are added and incubated at room temperature for 5-30 minutes. Then, synoviolin, its active derivative or an active derivative of immobilized synoviolin is added to initiate auto-ubiquitination reaction of synoviolin. Incubation is carried out at a constant temperature in a range of room temperature to 37°C for a predetermined period in a range of 20-120 minutes, and a predetermined amount of a terminating buffer (containing 0.2 M boric acid buffer, TritonX-100 and EDTA) is added to terminate the reaction. The ubiquitin binding to and not binding to synoviolin are quantified to determine the degree of auto-ubiquitination.
(2) Applications to examination, diagnosis, and assessment of therapeutic effect or drug efficacy
   Synoviolin is strongly expressed in RA patients' synovial tissue. Antibody that recognizes synoviolin (autoantibody) is frequently detected in RA patients' blood. On the other hand, substantially no antibody against synoviolin can be detected in healthy person's blood. Furthermore, synoviolin inhibits proliferation of cultured synovial cells *in vitro.* It is contemplated that this is caused because synoviolin competes for the ligand that promotes proliferation of synovial cells. Based on this information, the following molecular mechanism can be assumed. Strong expression of synoviolin in the synovial cell promotes binding between synoviolin and the ligand having a proliferation promoting activity on the synovial cells, as a result of which proliferation of synovial cells is promoted. This abnormal proliferation of the synovial cells is literally the clinical condition of RA.
   Based on such a finding, the present invention provides a method for detecting or diagnosing a cell proliferative disease by utilizing the immunological property of SL-1 antibody. Examples of the cell proliferative diseases include but not limited to rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia and malignant lymphoma. These cancers may be either primary or metastatic. Examples of the cells include but not limited to synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell and apical ectodermal ridge.
   These methods comprise the steps of:
   (i) reacting a biological sample from a subject with the antibody of the invention to detect a marker of a disease present in the sample; and
   (ii) link the detection result obtained in step (i) with a disease.

   The marker may be any of the following markers (a) to (d). A method for determining these markers will be described later below.
   (a) Synoviolin or a protein functionally equivalent to synoviolin;
   (b) synoviolin or a peptide functionally equivalent to synoviolin;
   (c) antibody that binds to synoviolin or a protein functionally equivalent to synoviolin; and
   (d) antibody that binds to synoviolin or a peptide functionally equivalent to synoviolin.

   For example, if an antibody that reacts with synoviolin or a protein or a peptide functionally equivalent to synoviolin is found in a blood sample taken from a subject, that subject is likely to have RA.
   Alternatively, expression of synoviolin or a protein functionally equivalent to synoviolin in synovial tissue taken from a subject indicates proliferation of the synovial tissue caused by RA. In general, expression of a protein can be detected using, as an index, the presence of the protein itself or mRNA having information of the protein. In order to detect synoviolin, SL-1 antibody of the invention is preferably used. Detection of synoviolin in synovial cells, synovial tissue or body fluid possibly indicates progression ofRA.
   Other than for diagnosis ofRA, a reagent used for immunological analysis containing the antibody of the invention is also useful for assessing a therapeutic effect or a drug efficacy. Assessment of a therapeutic effect or a drug efficacy against RA is carried out basically in the same manner as the diagnosis of RA. The level of synoviolin or an antibody thereto in synovial tissue or blood relates to prevalence of expression of the gene coding for these proteins and detection of them serves as an index indicating transition of the conditions and remission of the disease.
   Moreover, SL-1 antibody of the invention can be used for separating or detecting cells expressing synoviolin. Examples of such cells include but not limited to synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell and apical ectodermal ridge. Expression of synoviolin is observed in apical ectodermal ridge upon emergence and synoviolin is strongly expressed in rheumatoid synovial cells and undifferentiated mesenchymal cells as primordia of synovial membrane, bone, cartilage and limbs. Thus, preferably, synoviolin is used as a marker particularly for apical ectodermal ridge, rheumatoid synovial cell and undifferentiated mesenchymal cell.
   Specifically, expression of synoviolin can be used as an index to detect or separate synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell or apical ectodermal ridge, especially apical ectodermal ridge, rheumatoid synovial cell or undifferentiated mesenchymal cell. For example, an anti-synoviolin monoclonal antibody against synoviolin is labeled with an appropriate fluorescence or the like and reacted with cells. Then, cells expressing synoviolin can be separated by cell sorting or the like. The separated undifferentiated mesenchymal cells are useful for *in vitro* or *in vivo* remodeling of tissue such as muscle, tendon, fat and stroma (e.g., bone marrow), bone and cartilage formation or joints. The remodeled tissue or organ is expected of its application in regenerative medicine as well as in fundamental studies.
   The antibody of the invention may also be used for producing an antibody column for purifying synoviolin and for detecting the protein present in each fraction upon purification. Of course, the use of the anti-synoviolin monoclonal antibody according to the invention is not limited to these applications.
(3) Analysis using reagent for immunological analysis
   Hereinafter, how a reagent for immunological analysis containing SL-1 antibody is used for the above applications will be specifically described.
   Many techniques are commonly employed as an immunological analysis method for an antigen or an antibody in a sample. A determination method using a monoclonal antibody against synoviolin or a fragment thereof is not limited to a particular method and can be any method as long as the method comprises detecting the quantity of antigen in the sample, i.e., the quantity of an antibody or an antigen-antibody complex corresponding to the quantity of synoviolin, by chemical or physical means and calculating the quantity based on a calibration curve generated with a standard solution containing a known quantity of antigen. Specifically, examples of preferable methods for detecting a quantity of antigen-antibody complex include a competitive method, an immunometric method, nephelometry and a sandwich method, while a sandwich method is particularly preferable from sensitivity and specificity perspectives.
   According to a sandwich method (e.g., ELISA method), a sample (containing synoviolin to be quantitated) is reacted with a solid-phased SL-1 antibody of the invention (primary reaction), subsequently reacted with a labeled anti-synoviolin antibody (secondary reaction) and then the activity of the labeling substance on the solid-phased carrier is determined to quantify the synoviolin in the sample. In this case, the order of the primary and secondary reactions may be reversed, and they may be performed simultaneously or with a time lag. The antibody used for the secondary reaction is preferably an antibody that binds to a different site of synoviolin from the site where the SL-1 antibody of the invention binds.
   According to an immunometric method using the monoclonal antibody of the invention, an antigen in a sample and a solid-phased antigen are made to compete for a predetermined amount of labeled monoclonal antibody, followed by separation between the solid phase and the liquid phase. Alternatively, an antigen in a sample and an excessive amount of labeled monoclonal antibody are reacted, a solid-phased antigen is added to bind unreacted labeled antibody to the solid phase, and then the solid phase and the liquid phase are separated. Thereafter, the amount of the label in either of the phases is determined to quantify the antigen in the sample.
   According to a competitive method, an antigen in a sample and a labeled antigen are made to compete for the antibody. The unreacted labeled antigen (F) is separated from the antibody-binding labeled antigen (B) (B/F separation) and the amount of the label of either B or F is determined to quantify the amount of antigen in the sample. An alternative method may be a liquid phase method in which a soluble antibody is used as the antibody, while polyethyleneglycol and a secondary antibody to the antigen are used for B/F separation, or a solid phase method in which the primary antibody is a solid-phased antibody or in which the primary antibody is a soluble antibody while the secondary antibody is a solid-phased antibody. For example, an anti-synoviolin monoclonal antibody of the invention solid phased on a carrier and a labeled antibody can simultaneously or successively be subjected to reaction to compete for a sample, and the activity of the labeling substance on the solid-phased carrier can be determined.
   According to nephelometry, the amount of insoluble precipitate resulting from antigen-antibody reaction is determined in a gel or in a solution. When only an extremely small amount of precipitate is obtained due to a small amount of antigen in the sample, laser nephelometry that utilizes scattering of laser is preferably employed.
   In order to detect and analyze the antibody in the sample, the most popular method as the immunological analysis method for an antibody is described below. The antibody in the sample reacts with antigen on the plate immunized with an antigen. Then the antibody for detection captured onto the surface of the plate is detected with the labeled antibody specifically recognized to the antibody in the sample as an antigen. (Immunochemistry, 8:871-879, 1971). Alternatively, SL-1 antibody of the invention may be used as a labeled antibody for detecting unbound antigen on the plate. A method in which antigen-adsorbed latex particles are mixed with a sample to detect the antibody as immunological agglutination is also known (Plotz C.M. and Singer J.M., Am. J. Med., 21:888-892, 1956). Immunological particle agglutination is a method that enables rapid analysis with a single reagent and is preferable for a large-scale screening.
   When SL-1 antibody of the invention is used as a reagent for separating or detecting cells, an anti-synoviolin monoclonal antibody may be combined with other solvent or solute to form a composition. For example, distilled water, a pH buffer reagent, salt, a protein, a surfactant or the like may be combined.
   A reaction reagent contains a label detectable with appropriate chemical or physical detecting means. A labeling substance used for such a detection method is, for example, a fluorescent substance, an enzyme, a radioisotope or a luminescent substance. In particular, a method that uses an enzyme as a label is called ELISA method and is used extensively.
   Examples of fluorescent substances include fluorescamine and fluorescein isothiocyanate; examples of enzymes include peroxidase, alkaline phosphatase, malate dehydrogenase, α-glucosidase and α-galactosidase; examples of radioisotopes include ¹²⁵I, ¹³¹I, ³H and ¹⁴C; and examples of luminescent substances include luciferin, lucigenin, luminol and luminol derivatives.
   A biotin-avidin system may be used for binding SL-1 antibody of the invention or an antigen with a labeling substance. The antigen or the monoclonal antibody may be solid phased by physical adsorption or a chemical bond method that is usually used for solid phasing or immobilizing a protein or an enzyme. As a carrier, a synthetic resin such as polystyrene, polyacrylamide and silicon, insoluble saccharides such as agarose, dextran and cellulose, or glass can be used.
   Examples of the reaction media include buffers that give optimum reaction conditions or that is useful for stabilization of the reaction-producing substance, or stabilizers for reaction substances.
   Detection means should be one that is capable of detecting the above labels, for example, a spectrometer, a radiation detector, light scattering detector or the like.
(4) Diagnostic kit
   No special condition or operation is required for applying SL-1 antibody of the invention to an immunological determination method. A preferable determination system can be designed with general conditions and operations according to each method, and if necessary, slight modifications can be added.
   In order to realize the most convenient and efficient determination, the above reagents are made into a kit. This kit will allow efficient quantification in a usual examining room or laboratory without the requirement of any special analysis apparatus, skilled operation or high knowledge. The component and embodiment of the assay kit for performing various diagnosis methods or methods for determining a therapeutic effect described above are not particularly limited as long as they can achieve a predetermined aim. Generally, a kit comprises an instruction for carrying out an assay for the sample described above and for interpreting the results, reaction reagents, a reaction medium in which reactions take place and a base material for providing a place of assay. If desired, the kit may further comprise a verification sample to be used as a reference for comparison or to produce a calibration curve as well as a detector.
(5) Pharmaceutical composition
   The antibody of the invention is useful as a pharmaceutical composition for treating or preventing cell proliferative diseases. Examples of cell proliferative diseases include but not limited to rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia and malignant lymphoma. These cancers may be either primary or metastatic.

Preferably, the pharmaceutical composition of the invention is provided in a form of a pharmaceutical composition that contains the antibody of the invention or a fragment thereof as an active ingredient and furthers a pharmaceutically acceptable carrier.

Herein, examples of "pharmaceutically acceptable carriers" include an excipient, a diluting agent, a filler, a disintegrating agent, a stabilizer, a preservative, a buffer, an emulsifying agent, an aromatic substance, a colorant, a sweetening agent, a thickening agent, a flavoring agent, a solubilizing agent or other additives. By using one or more such carriers, a pharmaceutical composition can be prepared in forms of an injectable agent, a liquid solution, a capsule, a suspension, an emulsion, syrup or the like. These pharmaceutical compositions may be administered orally or parenterally. Other form of a parenteral administration includes an injectable agent containing one or more active substance formulated by a conventional method.

Although a given dose of a drug of the invention differs depending on age, sex, weight and condition of the patient, a therapeutic effect, a method of administration, a treatment period or the type of the high affinity antibody as an active ingredient contained in the drug, a single dose is usually given in but not limited to a range of 600 µg to 6000 mg, preferably 6 to 600 mg for an adult.

For example, an injectable agent can be produced by dissolving or suspending the antibody in a pharmaceutically acceptable carrier such as physiological saline or commercially available injectable distilled water to a concentration of 1 mg antibody/ml carrier to 100 mg antibody/ml carrier. The injectable agent produced as such can be administered to a human patient in need of a treatment in 10 µg to 100 mg/kg weight, preferably 100 µg to 10 mg/kg weight for once to a few times a day. Examples of the administration forms include intravenous injection, subcutaneous injection, intradermal injection, intramuscular injection or intraperitoneal injection, preferably intravenous injection. As the case may be, the injectable agent may be prepared as a nonaqueous diluting agent (for example, propylene glycol, polyethyleneglycol, plant oil such as olive oil or alcohol such as ethanol), a suspending agent or an emulsifying agent. Sterilization of such an injectable agent may be performed by filter sterilization, blend of a disinfecting agent or the like. The injectable agent may be produced in a form that needs preparation upon use. Specifically, the injectable agent may be made into a sterile solid composition by freeze-drying or the like and may be used by dissolving in a sterile injectable distilled water or other solvent before use.

Hereinafter, the present invention will be described in more detail by way of examples. Although those skilled in the art can modify the present invention in various manners, the present invention is not limited to these examples. Unless otherwise stated, "%" refers to "% by weight".

### EXAMPLE 1

This example aims at preparing an anti-synoviolin monoclonal antibody.

In order to prepare an anti-synoviolin monoclonal antibody, three types of peptides containing the following partial amino acid sequences of human synoviolin were synthesized as peptides for immunization. These amino acid sequences were selected from regions that were presumed to have antigenicity:
Syno-P3 (SLALTGAVVAHAYYC/SEQ ID NO:3);
Syno-P2 (TCRMDVLRASLPAQS/SEQ ID NO:4); and
Syno-P1 (GAATTTAAGTSATAC/SEQ ID NO:5).

To each of the synthesized peptides, Keyhole Limpet hemocyanin (KLH) was bound via Cys in the amino acid sequence. Fifty µg each of the synthesized peptides bound to KLH was dissolved in 0.1 ml physiological saline and added with 0.1 ml of Freund's complete adjuvant (FCA) to prepare an immunogen. 0.2 ml each of the immunogens was subcutaneously injected to the back of eight mice (BALB/c female, 5 week old) for immunization. Immunizations were performed every two weeks for a total of four times and then once following another week. Eight days after the final immunization, blood was taken from the heart and serum was sorted for 200 µl or more. Spleen cells were taken from individuals whose antibody titers had increased as confirmed by ELISA, to perform cell fusion.

For each immunogen, antibody titers of mice sera from 3 individuals were determined by ELISA. For all of the immunogens, the individuals were confirmed to have increased antibody titers. All of these immunogens were found useful as immunogens of synoviolin.

Myeloma cell strain (P3U1) and the mouse spleen cell were mixed at 1:10 and fused in the presence of 50% PEG (Wako Pure Chemical Industries, PEG1540). Following cell fusion, the resultant was seeded onto a 96-well plate so that the number of the spleen cells was 5 x 10⁵/ml. After cultivation in a HAT medium for 10-14 days, proliferation of the cells was confirmed to examine the supernatant.

For the examination of the culture supernatant, ELISA plates having the respective synthesized peptides solid phased thereon were used. Examination was carried out as follows. After reacts the culture supernatant with the ELISA plate, positive wells were selected using anti-mouse IgG goat-peroxidase (POX). Wells to be subjected to cloning were selected and cells in other positive wells were preserved in a frozen state.

After a few days, the cells were seeded onto 96-well plates to 100 cells/plate (20 cells/ml) using one plate for each strain and cultured for 10-14 days. Colonies were confirmed and the culture supernatants were examined. The supernatants were examined by applying 50 µl of the supernatant to the screening ELISA plate having antigen solid phased thereon described above. For the secondary antibody, anti-mouse IgG goat-POX was used. The selected colonies were cultured, re-cloned, cultured for 10-14 days, and subjected to the same procedure of colony confirmation and examination of the culture supernatants. Wells were selected by parent strain and cultured in a 24-well plate. Supernatants were collected for checking the clones to examine antibody subclasses and antibody production. As a result of the cloning, clone SL-1 obtained by using Syno-P2 as the immunogen was selected as a hybridoma that highly efficiently produces a monoclonal antibody having high affinity to synoviolin.

### EXAMPLE 2

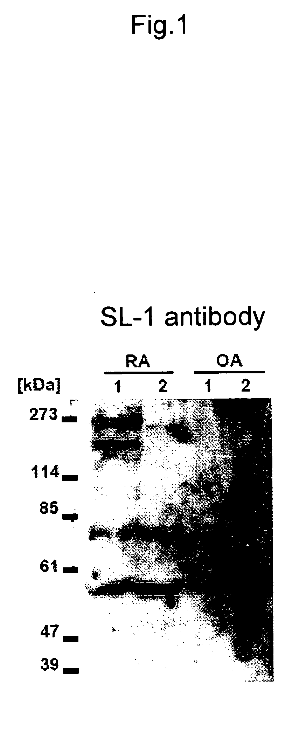

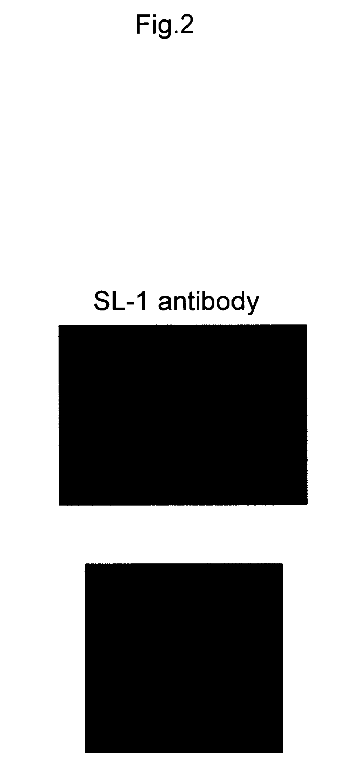

The present example aims at detecting synoviolin from a patient's sample using an anti-synoviolin monoclonal antibody.
(1) Western blotting of synovial cells derived from patient using anti-synoviolin monoclonal antibody
   A protein of synovial cell derived from a patient suffering from chronic rheumatic arthritis (RA) was separated by SDS-PAGE and subjected to western blotting using the SL-1 antibody that recognized Syno-P2 obtained in Example 1. In carrying out western blotting, the SL-1 antibody obtained in Example 1 was used as the antibody and anti-mouse IgG sheep-HRP was used as the labeled antibody.
   As a control, synovial cells derived from patients suffering from osteoarthritis (OA) were also analyzed. As a result, specific signal was detected in the synovial cells from the RA patients (Figure 1, bands near 85 kDa in lanes 1 and 2 of "RA"). The SL-1 antibody obtained in Example 1 was confirmed to recognize synoviolin more specifically.
(2) Fluorescent immunostaining of synovial cell from RA patient using anti-synoviolin monoclonal antibody
   SL-1 antibody was used to perform fluorescent immunocytochemical analysis of synovial cell from an RA patient. Immunostaining was carried out as described in Example 9 of WO 02/052007 pamphlet except that SL-1 antibody from Example 1 was used as the antibody and anti-mouse IgG sheep-FITC as the labeled antibody. Signal of synoviolin was strongly detected in the synovial cell from the RA patient (Figure 2, upper panel) but not detected in the control that was reacted with the secondary antibody only (Figure 2, lower panel).
(3) Immunostaining of synovial tissue from RA patient using anti-synoviolin monoclonal antibody

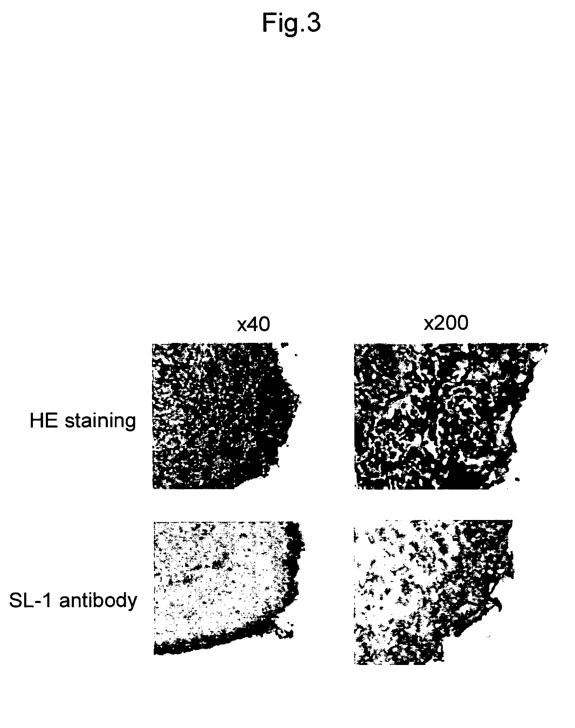

SL-1 antibody was used to perform immunostaining of a section of synovial tissue taken from an RA patient. Immunostaining was carried out as described in Example 9 of WO 02/052007 pamphlet except that SL-1 antibody from Example 1 was used as the antibody and anti-mouse IgG sheep-HRP as the labeled antibody. Synoviolin was strongly expressed in the synovial tissue from the RA patient (Figure 3, panels of SL-1 antibody). From HE staining that was performed at the same time, a layer of proliferated synovial cells was observed which was stained with the monoclonal antibody. From these results, the SL-1 antibody of the invention was confirmed to specifically recognize synoviolin in synovial tissue of an RA patient (Figure 3, panels of HE staining).

As described above, the anti-synoviolin antibody can be used to detect synoviolin in a patient's sample, thereby examining and diagnosing RA.

### EXAMPLE 3

This example aims at developing an ELISA test drug for detecting synoviolin.

An appropriate amount of SL-1 antibody obtained by the method in Example 1 was dissolved in PBS to 20 µg/ml. The solution was dispensed in a 96-well flat bottom microplate for enzyme immunoassay (EIA) for 100 µl/well and allowed to stand still for a few hours at room temperature. The solution was removed from the wells. The wells were washed with PBS containing 0.05(V/V)% Tween20, added with 100 µg/well of PBS containing 1 (V/V)% fetal bovine albumin (BFA) and allowed to stand still at 4°C overnight to block the monoclonal antibody. Then, PBS was removed to obtain an antibody plate.

Beside the above operation, a polyclonal antibody was prepared by immunosensitizing a rabbit with synoviolin according to a conventional method. This polyclonal antibody was labeled with horseradish peroxidase (HRP) by periodate oxidation and purified by gel filtration chromatography using Sephacryl S-300HR (Pharmacia) pre-equilibrated with PBS to obtain a labeled antibody. This secondary labeled antibody was a buffering reagent composition containing salt, stabilizer, antiseptic or the like.

To the 96-well microplate having solid-phased SL-1 antibody obtained above, a body fluid such as blood or urine or tissue of a patient that was appropriately diluted was dispensed and reacted for an hour at room temperature according to a normal EIA. A well added with only the diluting solution was used as a control. After washing with a phosphate buffer containing 0.05% Tween20, HRP-labeled anti-mouse IgG rabbit antibody was added to react for 30 minutes at room temperature. After the secondary labeled antibody that did not undergo the reaction was washed with a phosphate buffer containing 0.05% Tween20, 0.1M citrate buffer solution containing 0.015% hydrogen peroxide as a substrate buffer and o-phenylenediamine-citrate buffer solution (10 mg/ml) as a staining agent were added to each well and reacted for 30 minutes at room temperature. The chromogenic reaction was terminated by adding 2M sulfuric acid. Then, color was identified by determining the absorbance at 492 nm with a microplate reader to quantify synoviolin in the sample.

### EXAMPLE 4

This example aims at examining auto-ubiquitination of synoviolin.

Synoviolin is E3 ubiquitin-protein ligase having a RING finger motif, which is believed to be a binding site for E2 ubiquitin-conjugating enzyme. The E3 ubiquitin-protein ligase is known to cause auto-ubiquitination and has also been empirically confirmed to be associated with auto-ubiquitination of synoviolin.

Mouse monoclonal antibody SL-1 was prepared using as an antigen a peptide corresponding to amino acids 328-342 of the RING finger domain of synoviolin protein (Example 1). His-tagged fusion protein MBP-dTM Syno-His was used, mixed with SL-1 antibody, anti-FLAG antibody or mouse IgG at a predetermined concentration for antigen-antibody reaction at 4°C for 1.5 hours.

Thereafter, auto-ubiquitination of synoviolin was examined using an *in vitro* ubiquitination reaction system. E1 (derived from yeast), E2 (UbcH5c), ATP, GST-HA-ubiquitin and each of the MBP-dTM Syno-His pre-reacted with the antibody were mixed and reacted at 37°C for 120 minutes. Following reaction, each sample was added with 4 x SDS-PAGE buffer, boiled for 5 minutes and separated on 10% SDS-PAGE. A band of ubiquitinated synoviolin was detected using SL-1 antibody according to a western blotting analysis.

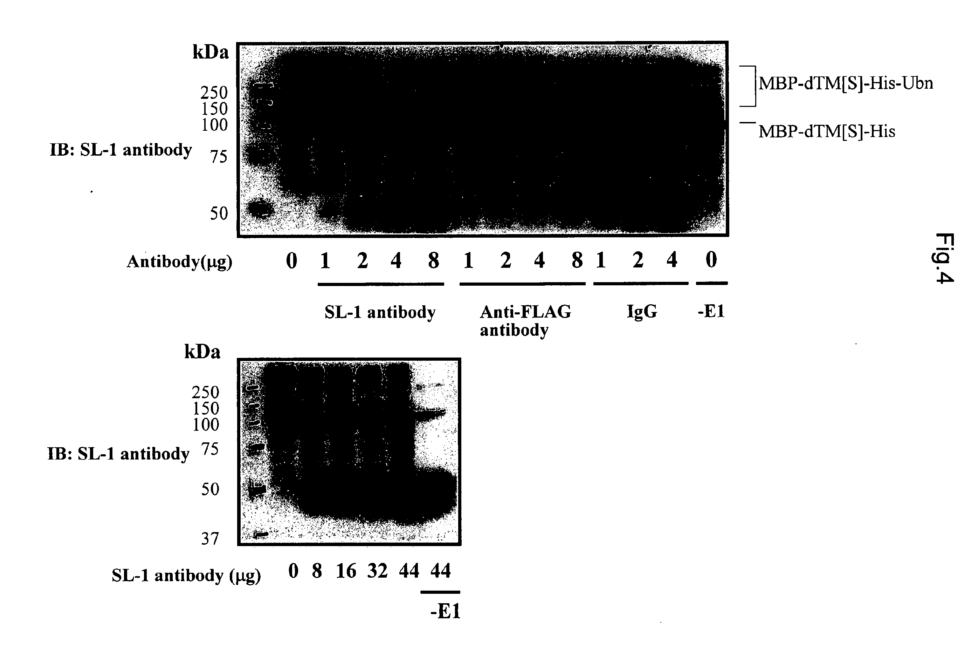

As a result, since the band at 250 kDa was inhibited depending on the amount of SL-1 antibody, it was observed that auto-ubiquitination of MBP-dTM Syno-His was inhibited with 2 µg SL-1 antibody. The anti-FLAG antibody or the mouse IgG of the same amount, however, did not show activity of inhibiting the band at 250 kDa (Figure 4, upper panel). The band near 250 kDa was also inhibited in the same experiment with different amounts of SL-1 antibody ranging from 8 to 44 µg/L, indicating that auto-ubiquitination of MBP-dTM Syno-His was inhibited with SL-1 antibody (Figure 4, lower panel). Thus, auto-ubiquitination of MBP-dTM Syno-His was proven to be inhibited specifically with SL-1 antibody.

### EXAMPLE 5

This example aims at examining whether SL-1 antibody also influences ubiquitination of synoviolin.

Ubiquitin covalently binds to a target protein (substrate) via a ubiquitin system including ubiquitin-activating enzyme (E1), ubiquitin-conjugating enzyme (E2) and ubiquitin ligase (E3). A polyubiquitin chain that is formed by repetition of this reaction serves as a marker for proteasome degradation of the substrate. P4HA1, an a-subunit of prolyl hydroxylase, an enzyme that catalyzes hydroxylation of a proline residue of collagen essential for collagen production, has been found as a substrate of synoviolin. Since SL-1 antibody has been found to inhibit auto-ubiquitination activity of synoviolin, we also examined whether this antibody also influences ubiquitination of synoviolin by P4HA1.

First, MBP-dTM Syno-His was mixed with a predetermined concentration of SL-1 antibody, anti-FLAG antibody or mouse IgG for antigen-antibody reaction at 4°C for 1.5 hours. Then, in an *in vitro* ubiquitination reaction system, 2 µg of GST-fused P4HA1 protein GST-P4HA1 was mixed with E1 (derived from yeast), E2 (UbcH5c), ATP, GST-HA-ubiquitin and each of the MBP-dTM Syno-His pre-reacted with the antibody were mixed and reacted at 37°C for 120 minutes. Following reaction, each sample was added with 4 x SDS-PAGE buffer, boiled for 5 minutes and separated on 10% SDS-PAGE. A band of ubiquitinated P4HA1 was detected using anti-GST antibody according to western blotting analysis.

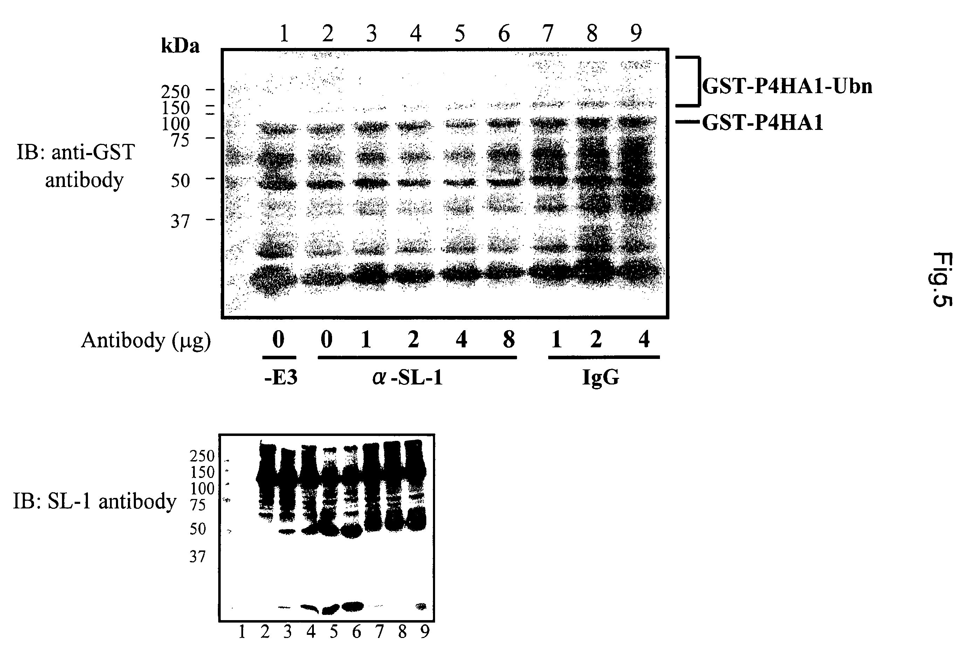

As a result, although auto-ubiquitination of MBP-dTM Syno-His was clearly inhibited with 4 µg SL-1 antibody, ubiquitination of GST-P4HA1 was found uninhibited even with 8 µg of SL-1 antibody (Figure 5). In Figure 5, -E3, α-SL1 and IgG represent a synoviolin-free sample, an antibody-added sample and a negative control sample, respectively, and a band near 250 kDa shown as GST-P4HA1-Ubn indicates ubiquitinated GST-P4HA1. Thus, it was found that SL-1 antibody specifically inhibits auto-ubiquitination of synoviolin but does not influence ubiquitination of GST-P4HA1.

### INDUSTRIAL APPLICABILITY

An antibody of the invention can regulate auto-ubiquitination of synoviolin.

Furthermore, finding of an autoantibody that recognizes synoviolin in the blood of an RA patient brings a totally new approach to diagnosis of RA. Thus, a pharmaceutical composition containing the antibody of the invention can propose a new unprecedented approach to the development of an RA treatment.

The present invention also provides a kit for detecting a cell containing synoviolin by utilizing the above antibody-producing cell and the above monoclonal antibody.

A synoviolin gene involved in developments of synovial membrane, bone, cartilage and limbs is also involved in clinical conditions of RA and an antibody against this gene product is produced in RA patients. SL-1 antibody of the invention can be utilized for specifically detecting and quantifying synoviolin as a marker useful for RA diagnosis as well as the antibody thereto frequently found in patient's serum, thereby contributing to diagnosis of RA diseases and determination of a therapeutic effect.

With its high specificity, SL-1 antibody of the invention is capable of collecting undifferentiated mesenchymal cell from embryo cell or the like using synoviolin as a cell marker, thereby contributing to application in regenerative medicine.

## Claims

1. An antibody or a fragment thereof against synoviolin, which is capable of inhibiting auto-ubiquitination of synoviolin.

2. An antibody or a fragment thereof according to Claim 1, wherein it does not influence ubiquitination of a substrate protein of the synoviolin.

3. An antibody or a fragment thereof according to either one of Claims 1 or 2, wherein the antibody is a monoclonal antibody.

4. A monoclonal antibody against synoviolin or a fragment thereof produced by a hybridoma obtained by cell fusion between a myeloma cell and an antibody-producing cell derived from an animal immunized with a peptide having an amino acid sequence represented by any one of SEQ ID NOS:3-5 as an antigen, which is capable of inhibiting auto-ubiquitination of synoviolin.

5. A hybridoma obtained by cell fusion between a myeloma cell and an antibody-producing cell derived from an animal immunized with a peptide having an amino acid sequence represented by any one of SEQ ID NOS:3-5 as an antigen, which produces a monoclonal antibody capable of inhibiting auto-ubiquitination of synoviolin.

6. A method for producing a monoclonal antibody against synoviolin capable of inhibiting auto-ubiquitination of synoviolin, comprising: culturing a fusion cell between a myeloma cell and an antibody-producing cell derived from an animal immunized with a peptide having an amino acid sequence represented by any one of SEQ ID NOS: 3-5 as an antigen; and collecting the monoclonal antibody from the resulting culture.

7. A pharmaceutical composition comprising an antibody or a fragment thereof according to any one of Claims 1 to 4.

8. A pharmaceutical composition according to Claim 7 for treating or preventing rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia or malignant lymphoma.

9. An inhibitor for auto-ubiquitination of synoviolin, comprising an antibody or a fragment thereof according to any one of Claims 1 to 4.

10. A reagent for detecting a cell containing synoviolin, comprising an antibody or a fragment thereof according to any one of Claims 1 to 4.

11. A reagent for detecting a cell proliferative disease caused by synoviolin, comprising an antibody or a fragment thereof according to any one of Claims 1 to 4.

12. A reagent according to Claim 11, wherein the cell proliferative disease is at least one selected from the group consisting of rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia and malignant lymphoma.

13. A reagent according to any one of Claims 10 to 12, wherein the cell is any cell selected from the group consisting of synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell and apical ectodermal ridge.

14. A method for inhibiting auto-ubiquitination of synoviolin, comprising reacting an antibody or a fragment thereof according to any one of Claims 1 to 4 with synoviolin.

15. A method for detecting a synoviolin-expressing cell, comprising reacting an antibody or a fragment thereof according to any one of Claims 1 to 4 with a biologic sample.

16. A method for detecting a cell proliferative disease caused by synoviolin, comprising reacting an antibody or a fragment thereof according to any one of Claims 1 to 4 with a biologic sample taken from a subject.

17. A method according to Claim 16, wherein the cell proliferative disease is at least one selected from the group consisting of rheumatic arthritis, cancer, fibrosis, arteriosclerosis, Castleman's disease, multiple myeloma, Crohn's disease, systemic juvenile idiopathic arthritis, brain tumor, tongue cancer, pharynx cancer, lung cancer, breast cancer, esophageal cancer, gastric cancer, pancreas cancer, biliary tract cancer, gallbladder cancer, duodenal cancer, colon cancer, liver cancer, uterus cancer, ovary cancer, prostate cancer, kidney cancer, bladder cancer, rhabdomyosarcoma, fibrosarcoma, osteosarcoma, chondrosarcoma, skin cancer, acute myeloid leukemia, acute lymphoid leukemia, chronic lymphoid leukemia, adult T-cell leukemia and malignant lymphoma.

18. A method according to any one of Claims 15-17, wherein the cell is any cell selected from the group consisting of synovial cell, osteoclastic cell, keratinized epithelial cell, blood cell, cancer cell, bone-marrow cell, fibroblast, vascular endothelial cell, dermal cell, muscular cell, nerve cell, lymph cell, vascular smooth muscle cell, hepatic cell, pigment cell, fat cell, uterine endothelial cell, alveolar epithelial cell, undifferentiated mesenchymal cell and apical ectodermal ridge.
